# EUROPEAN PATENT SPECIFICATION

(11) **EP 1 471 789 B1**
(45) Date of publication and mention of the grant of the patent: **15.04.2009**
(21) Application number: 02710268.0
(22) Date of filing: 05.02.2002
(51) Int. Cl.: A01N 65/00

(54) **NOVEL COMPOSITION FOR EARLY AND PROFUSE SPORULATION IN FUNGI AND A METHOD THEREOF**
NEUE ZUSAMMENSETZUNG UND METHODE FÜR DIE FRÜHE UND ÜPPIGE PILZ-SPORULATION
NOUVELLE COMPOSITION FAVORISANT LA SPORULATION PRECOCE ET PROFUSE DE CHAMPIGNONS, ET PROCEDE ASSOCIE

(43) Date of publication of application: 03.11.2004
(73) Proprietor: Council of Scientific and Industrial Research, New Delhi 110 001 (IN)
(72) Inventor: KHAJURIA, Rajinder, Kumar, 180 001 Jammu (IN); SINHA, Ram, Vilas, Parsad, 180 001 Jammu (IN); VERMA, Vijeshwar, 180 001 Jammu (IN); QAZI, Ghulam, Nabi, 180 001 Jammu (IN); HANDA, Sukhdev, Swami, 180 001 Jammu (IN)
(74) Representative: Fairbairn, Angus Chisholm
(86) International application number: PCT/IB2002/000438
(87) International publication number: WO 2003/065812

(56) References cited:
- LEWIS, J.A.; PAPAVIZAS, G.C.: "Production of chlamydospores and conidia by Trichoderma-spp in liquid and solid growth media" SOIL BIOL. BIOCHEM., vol. 15, no. 3, 1983, pages 351-357, XP001105206
- BASTOS, CLEBER N.: "Effect of temperature, pH and nutrition on growth and sporulation of Trichoderma stromaticum sp. nov., an antagonist of cocoa witches' broom pathogen" SUMMA PHYTHOPATOLOGICA, vol. 27, no. 1, 2001, pages 73-76, XP001105216
- JACKSON ET AL.: "Nutritional studies of four fungi with disease biocontrol potential" ENZYME MICROB. TECHNOL., vol. 13, no. 6, 1991, pages 456-461, XP001105492 cited in the application

## Description

### Technical Field

The present invention relates to a composition useful for early and profuse sporulation in fungi with about 100 fold increase in spore count within 48 hours of inoculation, said composition comprising molasses ranging between 5-20g/L, Corn Steep Liquor (CSL) ranging between 10-25g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄.7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent and a method thereof.

### Background Art

*Trichoderma* species are used as efficient biocontrol agents against the soil-borne Wilt and Root-rot diseases of various crop plants. With the growing awareness of the harmful effects of many synthetic chemical pesticides on humans, non-target animals and plant species, and the environment as a whole, there has been a shift in attention to the research and development of more environment-friendly methods of pest and disease control.

Use of environmentally safe Disease Management Technologies based on Biopesticide and Biocontrol agents through natural enemies - parasitoids, predators and pathogens in integrated pest management programmes are ever increasing. Biocontrol agents like *Trichoderma* species and *Gliocladium* species are examples of some of the biocontrol agents used in the management of root diseases of chickpea, peas, groundnut, soybean spices and vegetables.

Root-rot diseases of crops in general and pulses in particular are a serious problem in rain-fed areas caused mostly by species of *Fusarium and Rhizoctonia* etc. Chemical control of these diseases requires a continuous use of fungicides, which is not economical and causes deleterious effects on the rhizosphere microflora and also have the residual problems.

WHO now has banned even some of the commonly used fungicides due to their severe toxicity. Under these circumstances the biocontrol agents can be used for the management of Root-rot diseases at comparatively cheaper cost with long term antagonistic effects against the pathogens because these biocontrol agents multiply and remain near the root zone of the plant giving protection to these plants throughout the growth period.
Investigations carried out world over have confirmed, that synthetic plant protection chemicals such as fungicides, insecticides and herbicides deteriorate soil fertility. Such deterioration occurs through disappearance of bio-diversity in soils. On account of this, several strains of soil microorganisms have been used which are able to suppress a wide range of disease causing microorganisms.

Jackson et al., (Enzyme-Microb. Technol.; (1991) 13, 6, 456-61) reported the culture media optimisation for production of the biological control agents *Gliocladium virens* G20, *Trichoderma pseudokoningii* IMI 322662, and *T. viride* IMI 322659 and IMI 322663. In glucose-alanine medium, the optimum dry wt./g carbon occurred with a C:N ratio of 15:1. Addition of 3.28-mg atoms iron/l to the medium increased biomass production of all isolates, but a concentration of 164-mg atoms/l was toxic to the *Trichoderma* species. Growth decreased in media lacking Mg, P, K or S, but the amount of the decrease differed between the four isolates. Sporulation in agar was reduced in the absence of Mg, P, K and N. Addition of biotin, p-amino -benzoic acid and thiamine-HCl increased biomass production slightly. The glucose-alanine basal medium supported better growth of all 4 isolates than a commercial molasses-yeast medium; conidia production was greater in the molasses-yeast medium. The pH of the glucose-alanine medium remained constant at 4.5, whereas the pH of the yeast-molasses medium (initially 5.5) increased to 8.0-8.6. Chlamydospores were produced by all isolates, but the numbers varied according to the culture conditions used.

Gervais and Sarrette ( J.Fement.Bioeng. (1990, 69, 1, 46-50) reported the Emerson agar medium (1 8 g/l agar) containing sodium octanoate (1 g/1) as a 2-heptanone precursor and glycerol as a water activity depressor for the solid-state fermentation of *T*. *viride* for cheese aroma production. Cultures were grown in Petri dishes at 20 °C. Sporulation was visible on the 9th day.

Toyama et al (J. Ferment. Technol.; (1983, 61, 4, 409-11) reported a sporulation medium for *T*. *reesei* QM 9414, to be used for the production of protoplasts. Kennedy-M-J; Davies-R-J; Surrey-M-R; Reader-S-L; Hoefakker-P-C, Australia's. Biotechnol; (1995) 5, 6, 349-54 reported preparation of biological control agents based on *Serratia entomophila, Bacillus thuringiensis, Pestalotia* species, *Truncatella augustata, Trichoderma viride, Trichoderma* species., cricket-paralysis Virus, flock-house virus. The report focuses on the 4 areas of process expertise required to develop and produce a commercial biological control agent: (a) culture medium design, (b) scale-up of the biological production system, (c) downstream processing of the product and (d) extending the shelf life of the biological control agent.

Nigam P. (Process-Biochem.; (1994, 29, 5, 337-42) reported a medium containing diluted molasses solutions of 3-4% sugar concentration for *T. viride* QM 9414, *T. reesei* Rut-C-30 NRRL 11460 under submerged fermentation conditions (SF) performed in flasks in 100 ml medium with agitation at 180 rpm for 5 days.

Culture media for two-stage culture of *T. longibrachiatum, T*. *viride, T*. *aureoviride,* AN: 91-13819, CA: Moscow-Tech.lnst.Food-Ind. were reported.

Abou-Zeid (Biorcsource-Technol.; (1991) 37, 3, 239-42) reported the following medium (pH 6) (g/l): cellulosic-source, 10.0; (NH₄)₂S0₄, 2.0; KH₂P0₄, 1.0; KCl, 0.5; MgS0₄.7H₂0, 0.5; MnSO₄.4H₂0, 0.05; and FeSO₄.7H₂0, 0.005, for the Fermentation of *T. viride* using leaflets and midribs of date palm (*Phoenix dactylifera*) leaves dried at 100-105 ° C, 144 hr at 30° C with 200 rpm agitation. Pre-treatment of palm leaflets with 1% NaOH and 5% H₂SO₄ improved their suitability as C-sources for *T. viride* growth.

The conventional media reported earlier are based on expensive gradients such as yeast extract, malt extract, protein hydrolysates and the like. Such a media are not suited for the economic mass production of these biocontrol agents by fermentation at technical scales.

Soil Biol. Biochem. (1983), 15(3), 351-357 discloses methods for sporulation of *Trichoderma spp* in liquid and solid media, comprising compositions containing molasses, corn steep liquor, and sodium chloride. The prepared media were inoculated with 1 ml of conidial suspension containing 1x10³ spores/ml. Cultures were incubated for 3 weeks at 25°C, and the influence of pH and agitation were also observed. At the end of the incubation period, *chlamydospores* and *conida* could be found in concentrations between 10⁸ and 10¹⁰ spores/g.

We have designed medium for the mass production of spores of *Trichoderma* species, a number of other sporulating ascomycetous fungi such as *Aspergillus* species, *Penicillium* species and other scantily sporulating species of fungi under solid state, surface and submerged fermentation conditions based on inexpensive agro-based industrial by-products like beet-root pulp, wheat bran, corn-steep liquor and molasses. These agro-waste industrial by products are rich in micro-nutrients and other essential elements like sugars, amino acids providing synergistic effects on the growth and sporulation of these fungi.

### Disclosure of the invention

The main object of the present invention is to develop a composition for early sporulation of the fungi.

Another main object of the present invention is to develop a composition for profuse sporulation of the fungi.

Yet another object of the present invention is to develop a composition for early and profuse sporulation in *Trichoderma* species, *Aspergillus* species, and *Penicillium* species.

Still another object of the present invention is to develop a composition for early and profuse sporulation in scantily sporulating fungi.

Still another object of the present invention is to develop a composition much more economical than commercially available compositions.

Still another object of the present invention is to develop a use for early and profuse sporulation in fungi.

Still another object of the present invention is to develop a use for sporulation under solid state, surface, or submerged fermentation conditions.

### Summary of the present invention

The present invention relates to a composition useful for early and profuse sporulation in fungi with about 100 fold increase in spore count within 48 hours of inoculation, said composition comprising molasses ranging between 5-20g/L, corn Steep Liquor (CSL) ranging between 10-25g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄.7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent and a method thereof.

### Detailed description of the present invention

Accordingly, the present invention relates to a composition useful for early and profuse spoliation in fungi, said composition comprising molasses ranging between 5-20g/L, Corn Steep Liquor (CSL) ranging between 10-25g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄.7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent.

In an embodiment of the present invention, said composition comprises molasses in an amount of 7.50 g/L, Corn Steep Liquor (CSL) in an amount of 20 g/L, Sodium chloride (NaCl) in an amount of 10 g/L, calcium sulphate (CaSO₄) in an amount of 0.25 g/L, Potassium dihydrogen phosphate (KH₂PO₄) in an amount of 0.0060 g/L, Magnesium sulphate (MgSO₄.7 H₂O) in an amount of 0.0050 g/L, Copper sulphate (CuSO₄) in an amount of 0.0010 g/L, Ferrous sulphate (FeSO₄) in an amount of 0.0016 g/L, an anti-foam agent, and optionally solidifying agent.

In yet another embodiment of the present invention, silicon oil is used as an anti-foam agent.

In still another embodiment off the present invention, the silicon oil ranges between 0.1-0.5 ml.

In still another embodiment of the present invention, agar is used as a solidifying agent.

In still another embodiment of the present invention, the agar ranges between 5-25 g/L.

In still another embodiment of the present invention, said composition is used for mass production of fungi of biocontrol activity.

In a further embodiment of the present invention a use is provided of a composition for early and profuse sporulation in fungi with about 100 fold increase in spore count within 48 hours of inoculation, said use comprising:
(a) inoculating about one week old spore culture of the fungi in a medium comprising molasses ranging between 5-20g/L, Corn Steep Liquor (CSL) ranging between 10-25g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄.7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent,
(b) fermenting the inoculated culture at temperature about 27-30°C, and
(c) obtaining a large quantity of viable spores.

In still another embodiment of the present invention, said culture of step (a) is inoculated with said spores of final concentration of 1x10⁸ conidia/ml.

In still another embodiment of the present invention, said culture of step (b) is fermented under solid state, surface, or submerged fermentation conditions.

In still another embodiment of the present invention, said inoculated culture is rotated in a rotor at a rate of 200 to 400 revolutions/minute.

In still another embodiment of the present invention, said culture is agitated with said rotor at 1.5-3.5cm throw.

In still another embodiment of the present invention, said culture is fermented with a pressure of 4.8-9.6 kPag (0.7-1.4 psig).

In still another embodiment of the present invention, said culture is mixed with a carrier in a ratio ranging between 1:2 to 2:1, to develop a commercial formulation with biocontrol activity.

In still another embodiment of the present invention, the carrier used is lignite.

In still another embodiment of the present invention, said medium comprises molasses 7.50 g/L, Corn Steep Liquor (CSL) in an amount of 20 g/L, Sodium chloride (NaCl) in an amount of 10 g/L, calcium sulphate (CaSO₄) in an amount of 0.25 g/L, Potassium dihydrogen phosphate (KH₂PO₄) in an amount of 0.0060 g/L, Magnesium sulphate (MgSO₄.7 H₂O) in an amount of 0.0050 g/L, Copper sulphate (CuSO₄) in an amount of 0.0010 g/L, Ferrous sulphate (FeSO₄) in an amount of 0.0016 g/L, an anti-foam agent, and optionally solidifying agent.

The present invention can provide a duration of viability of spores of about 10-14 months at temperature of about 4°C with carriers.

The present invention can provide a duration of viability of spores of about 4-8 months at temperature of about 30°C with carriers.

The present invention can show an average spore count ranging between 1-5x10⁸ conidiospores/ml.

The present invention can provide development of a novel medium based on inexpensive agro-based industrial by-products like beet-root pulp, wheat bran, corn-steep liquor, molasses and some inorganic salts in a definite proportion useful for the production of viable spores of *Trichoderma* species (used as biocontrol agents) and a number of other sporulating ascomycetous fungi such as *Aspergillus* species, *Penicillium* species and other scantily sporulating species of fungi under solid state, surface and submerged fermentation conditions.

Conventional processes take 5-9 days for the production of 10 fold increase in the number of spores of *Trichoderma* species from the initial inoculum as against in the present novel medium wherein 100 fold increase in the number of spores is achieved in 48 hours.

This therefore makes the present invented process economical, fast and highly advantageous from the commercial viewpoint.

Ingredients based on agro-waste industrial by-products are high energy and protein rich-natural sources containing large number of trace elements and are very often used in culture media. However, these ingredients have not been used for inducing early sporulation in ascomycetous fungi. These ingredients in the medium result in very fast and better sporulation under submerged fermentation conditions when compared to conventional medium (Molasses-yeast extract medium) containing yeast extract, peptone, malt extract and refined sugars like glucose, & sucrose.

The above ingredients can be dissolved in de-ionised water and steam sterilised at 121°C / 15 lb for 15 min. The sterilisation of the medium is done *ex-situ* for the operation of small bench fermentors and *in-situ* when large vessels are operated.

For initiation of the fermentation process, spores from a week old culture of *Trichoderma* species raised on solid medium (FM1 medium with agar) can be eluted with pre-sterile Tween 80 solution (0.02% v/v in water) and inoculated in the fermentor to the final concentration of 1× 10⁶ conidia/ml.

Fermentation can be carried out in FM1 medium in cylindrical glass jars or stainless steel vessels at 29 ±1°C.

The vessels can be agitated with standard stirrers at a moderate rate of 200-400 rpm and aerated at a constant air flow of 0.5 to 1.0 volumes per volume of liquid per minute at 1 bar (kg) over pressure.

All the inoculated spores can germinate and vegetative growth of the fungus with a numerous chlamydospores can emerge within 24-h fermentation period.

Thereafter sporulation can set in and Chlamydospores can disappear completely within next 24-h fermentation period.

Fermentation can be terminated after an incubation period of 48-72 h or until the maximum conidiospore concentration of 1-5 x10⁸/ml is obtained and no chlamydospores are spotted under microscopic examination.

Whole culture broth can thereafter be thoroughly mixed with pre-sterilised lignite (dry heated at 120° C for 6-8 h. and later cooled to room temperature) of mesh size 204-250 in the ratio of 1: 2 and packed in 200 g packets in milky white pollywogs (size 6"x10") of 50-75 µg thickness.

The biocontrol formulation thus prepared can be stored at 4°C for a period of 12 months and at 30°C upto 6 months, without significant loss of viability.

It is surprising to notice sporulation of this exorbitant quantum in fungi and that too in such a short span of time. The said composition shows about 100-fold increase in spore count within 48 hours of inoculation. All the ingredients of the composition comprising molasses ranging between 5-20g/L, Corn Steep Liquor (CSL) ranging between 10-25g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄.7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent, show synergy to bring about unexpected early and profuse sporulation.

The said ingredients and their quantum in said composition are critical. Any change in the ingredients or their quantity do not produce desired results. In one experiment, molasses was not added in the said composition and sporulation was observed with only about 5-fold increase. Similarly, not adding corn-steep liquor led to only about 4-fold increase in the sporulation. Also, when all the said ingredients of the said composition are added but with potassium dihydrogen phosphate at concentration higher then the above-mentioned range, sporulation was only about 2-fold. Similarly, higher then the above defined range of copper sulphate showing normal (normal means using conventional substrate) sporulation with no increase in sporulation at all.

All these observations prove that all the said ingredients and their concentration ranges are extremely critical for the desired results. Any deviation from the same severely affects the sporulation in fungi.

The Following examples are given by way of illustration and should not construe the scope of the invention.

### EXAMPLES

### Example 1:

### Production of spores of Trichoderma species in shake flasks using FM1 medium.

Strains of *Trichoderma* species were obtained from culture repository of Indian Agriculture Research Institute New Delhi. The cultures are maintained on standard Potato-dextrose medium (Potatoes 300 (g/l), glucose 20 (g/l) agar 17 (g/l)). For the purpose of mass propagation of its spores, the culture is pre-grown for a week in test tubes or large suitable containers like Roux bottles on FM1 medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), MgS0₄.7 H₂0 0.0050 (g/l), CuS0₄ 0.0010 (g/l), FeSO₄ 0.0016 (g/l), and additional Agar 15.00.

For the production of the spores of *Trichoderma* species at the shake flask scale, the culture is raised in an Erlenmeyer flask of 500 ml capacity containing 100 ml of above described FM1 medium without agar, autoclaved at 121°C /15 lb for 15 min. The medium was inoculated with the spores of *Trichoderma* species with final concentration of 1x 10⁶ conidia /ml from one week old slant eluted in pre-sterile Tween 80 (0.02 % v/v in water) solution and incubated on rotary shaker agitated at 180 revolutions/ minute and 2.5 cm throw at 30°C for 4 days.

At the end of the fermentation time no vegetative cells or chlamydospores were seen under microscope. An average conidia count of 5x10⁸/ml broth was obtained. The viability of spores as determined by colony forming units on standard Potato Dextrose Agar (PDA) was of the order of 95 to 100 %. Spore count (5x 10⁸ /ml) is achieved in this novel medium is 100 times from the initial inoculum during the above incubation period.

On the other hand in conventional medium (Molasses-yeast extract medium containing Molasses 7.50 (g/l), Glycerin 7.50 (g/l), Sodium Chloride 10.00 (g/l), Yeast Extract 5.00 (g/l), CaSO₄ 0.25 (g/l), KH₂PO₄ 0.006 (g/l), M_{g}S0₄.7 H₂O 0.005 (g/l), CuS0₄ 0.001 (g/l), and Trace Elements 0.0016 (g/l)), it is only five fold during the same period. Besides, the number of chlamydospores in the conventional medium is much higher at any given time as compared to the new medium. Chlamydospores are not considered useful for packaging the fungi and only the conidiospores are used for downstream processing due to the better viability of the latter. The higher number of Chlamydospores in the conventional medium (Molasses-yeast extract) make it much inferior compared to the designed novel medium (FM1).

The viable conidia thus generated under submerged conditions are mixed with pre-sterilised lignite of 200 - 250 mesh size by doing so the spores remain viable for 6-8 months at 30°C. This lignite carrier based spores of *Trichoderma* species preparation was field tested on several crops with satisfactory results.

### Example 2:

### Production of spores of Trichoderma species in shake flasks using FM1 medium with higher concentration of ingredients.

The seed of spores of *Trichoderma* species was raised in Erlenmeyer flasks in FM1 medium (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), M_{g}S0₄.7 H₂O 0.0050 (g/l), CuS0₄ 0.0010 (g/l), FeSO₄ 0.0016 (g/l)) as described in example 1.

For the production of spores of *Trichoderma* species at the shake flask scale, the culture is raised in an Erlenmeyer flask of 500 ml capacity containing 100 ml of FM1 medium with following ingredients Molasses 20.00 (g/l), Corn Steep Liquor (CSL) 25.00 (g/l), Sodium chloride 15.00 (g/l), CaSO₄ 0.500 (g/l), KH₂PO₄ 0.01 (g/l), M_{g}S0₄.7 H₂O 0.01 (g/l), CuS0₄ 0.0050 (g/l), FeSO₄ 0.005 (g/l)) without agar, autoclaved at 121°C /15 lb for 15 min.

The medium was inoculated with the spores of *Trichoderma* species with final concentration of 1x 10⁶ conidia /ml from one week old slant eluted in pre-sterile Tween 80 (0.02 % v/v in water) solution and incubated on rotary shaker and agitated at 180 revolutions per minute and 2.5 cm throw at 30°C for 4 days.

At the end of the fermentation time no vegetative cells or chlamydospores were seen under microscope. An average conidia count of 5x 10⁸ /ml broth was obtained. The viability of spores as determined by colony forming units on standard Potato Dextrose Agar (PDA) was of the order of 92 to 100 %.

In the FM1 medium (with higher concentrations of ingredients) spore count (5x 10⁸ /ml) is achieved which is also 100 times from the initial inoculum during the above incubation period as against the conventional medium (Molasses-yeast extract medium containing Molasses 7.50 (g/l), Glycerin 7.50 (g/l), Sodium Chloride 10.00 (g/l), Yeast Extract 5.00 (g/l), CaSO₄ 0.25 (g/l), KH₂PO₄ 0.006 (g/l), M_{g}S0₄.7 H₂O 0.005 (g/l), CuS0₄ 0.001 (g/l), and Trace Elements 0.0016 (g/l)).

Only five-fold increase in the number of spores from the initial inoculum is achieved in the Molasses-yeast extract medium during the same incubation period. Besides, the number of chlamydospores in this medium is much higher at any given time as compared to the new medium.

However, the spore concentration per ml and incubation period remained the same in both FM1 medium with higher concentrations of ingredients and FM1 medium with lesser concentration of ingredients (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), M_{g}S0₄.7 H₂O 0.0050 (g/l), CuS0₄ 0.0010 (g/l), FeSO₄ 0.0016 (g/l).

### Example 3:

### Production of spores of Trichoderma species in 7 litre batch fermentor using the FM1 medium.

The seed was raised in Erlenmeyer flasks in FM1 medium (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), MgSO₄.7 H₂O 0.0050 (g/l), CuS0₄ 0.0010 (g/l), FeSO₄ 0.0016 (g/l) as described in example 1. 7 litres of FM1 medium (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), M_{g}S0₄.7 H₂O 0.0050 (g/l), CuS0₄ 0.0010 (g/l), FeSO₄ 0.0016 (g/l) along with 3ml of silicon oil as antifoam agent was sterilised *in-situ* in a 10 1 fermentor (New Brunswick, USA) at 121°C /15 lb. for 15 min. The medium on cooling was inoculated with 100 ml of freshly grown seed culture with the final spores concentration of 2 -3 x 10⁶.

The fermentor was set with the following parameters:

| | |
|---|---|
| Agitation | = 250 rpm |
| Aeration rate | = 0.5 vvm (v/v min⁻¹) |
| Temperature | = 29°C ± I |
| Pressure | = 8.3 kPag (1.2 psig) |

The fermentation was continued for 72 hours until the spore concentration of 1.0 x 10⁹ was achieved (Table 1). Data of periodic sampling for spore counting at regular intervals of time using both FM1 and conventional medium (Molasses-yeast extract medium containing: Molasses 7.50 (g/l), Glycerin 7.50 (g/l), Sodium Chloride 10.00 (g/l), Yeast Extract 5.00 (g/l), CaSO₄ 0.25 (g/l), KH₂PO₄ 0.006 (g/l), M_{g}S0₄.7 H₂O 0.005 (g/l), CuS0₄ 0.001 (g/l), and Trace Elements 0.0016 (g/l)) media are shown in the Tables 1&2.

**Table 1:**

| **Growth and sporulation** of *Trichoderma* species **in 7L fermentor using novel FM1 medium** | | | |
|---|---|---|---|
| **Time (h)** | **Conidiospores /ml** | **Chlamydospore s/ml** | **Dry biomass (g% w/v)** |
| 0 | 2.0x10⁶ | Nil | - |
| 12 | - | | 0.24 |
| 24 | 2.0x10⁷ | 1.2x10³ | 0.43 |
| 48 | 3.0x10⁸ | - | 0.31 |

**Table 2:**

| **Growth and sporulation** of *Trichoderma* species **in 7L fermentor using conventional (Molasses- yeast extract) medium** | | | |
|---|---|---|---|
| **Time (h)** | **Conidiospores/ ml** | **Chlamydospore s/ml** | **Dry biomass (g% w/v)** |
| 0 | 2x10⁶ | Nil | 0.05 |
| 24 | 0.25x10⁵ | 4.75x10⁵ | 0.44 |
| 48 | 7.3x10⁶ | 9.15x10⁶ | 0.45 |
| 72 | 1.05x10⁷ | 1.05x10⁷ | 0.50 |
| 96 | 0.95x10⁷ | 0.72x10⁷ | 0.45 |
| 120 | 2.0x10⁷ | 1.0x10⁷ | 0.45 |
| 144 | 5.0x10⁷ | 0.7x10⁷ | 0.35 |

This novel medium (FM1) reduces fermentation period from 72 hrs to 48 hrs for sporulation. Moreover spore count (3.0x10⁸) is achieved in this novel medium which is 100 times from the initial inoculum in 48 hrs whereas in case of conventional medium (Molasses-yeast) it is less than 10 times (1.05x10⁷ ) 72 hrs. Besides, number of chlamydospores in conventional medium is much higher as compared to the novel medium as any fixed time internal.

### Example 4:

### Production of spores of Trichoderma species in 7 litre batch fermentor using FM1 medium with higher concentration of ingredients.

The seed was raised in Erlenmeyer flasks in FM1 medium (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), M_{g}SO₄.7 H₂O 0.0050 (g/l), CuSO₄ 0.0010 (g/l), FeSO₄ 0.0016 (g/l) as described in example 1. 7 liters of FM1 medium with following ingredients Molasses 20 (g/l), Corn Steep Liquor (CSL) 25.00 (g/l), Sodium chloride 15.00 (g/l), CaSO₄ 0.500 (g/l), KH₂PO₄ 0.01 (g/l), M_{g}SO₄.7 H₂O 0.01 (g/l), CuS0₄ 0.0050 (g/l), FeSO₄ 0.005 (g/l) without agar, along with 3ml of silicon oil as autifoam was sterilised *in-situ* in a 10 1 fermentor (New Brunswick, USA) at 121°C /15 lb. for 15 min. The medium on cooling was inoculated with 1.00 ml of seed culture with the final spores concentration of 2-3 x 10⁶.

The fermentor was set with the following parameters:

| | |
|---|---|
| Agitation | = 250 rpm |
| Aeration rate | = 0.5 vvm (v/v min⁻¹) |
| Temperature | = 29°C ± I |
| Pressure | = 8.3 kPag (1.2 psig) |

The fermentation was continued for 72 hours until the spore concentration of 1.0 x 10⁹ was achieved (Table 3). Data of periodic sampling for spore counting at regular intervals of time using both FM1 and conventional (Molasses-yeast extract medium containing Molasses 7.50 (g/l), Glycerin 7.50 (g/l), Sodium Chloride 10.00 (g/l), Yeast Extract 5.00 (g/l), CaSO₄ 0.25 (g/l), KH₂PO₄ 0.006 (g/l), M_{g}SO₄.7 H₂O 0.005 (g/l), CuS0₄ 0.001 (g/l), and Trace Elements 0.0016 (g/l) media are shown in the Tables 3&4.

**Table 3**

| **Growth and sporulation** of *Trichoderma* species **in 7L fermentor using novel FM1 medium with higher concentration of ingredients** | | | |
|---|---|---|---|
| **Time (h)** | **Conidiospores /ml** | **Chlamydospore s/ml** | **Dry biomass (g% w/v)** |
| 0 | 2.0x10⁶ | Nil | - |
| 12 | - | | 0.29 |
| 24 | 2.8x10⁷ | 1.35x10³ | 0.48 |
| 48 | 3.6x10⁸ | - | 0.39 |

**Table 4**

| **Growth and sporulation** of *Trichoderma* species **in 7L fermentor using conventional (Molasses- yeast extract) medium** | | | |
|---|---|---|---|
| **Time (h)** | **Conidiospores/ ml** | **Chlamydospore s/ml** | **Dry biomass (g% w/v)** |
| 0 | 2x10⁶ | Nil | 0.05 |
| 24 | 0.29x10⁵ | 4.85x10⁵ | 0.49 |
| 48 | 7.9x10⁶ | 9.25x10⁶ | 0.50 |
| 72 | 1.15x10⁷ | 1.15x10⁷ | 0.60 |
| 96 | 1.00x10⁷ | 0.92x10⁷ | 0.40 |
| 120 | 2.2x10⁷ | 1.2x10⁷ | 0.44 |
| 144 | 5.15x10⁷ | 0.9x10⁷ | 0.30 |

Perusal of the fermentation data show that the spore concentration/ml and incubation period remained the same as in the FM1 medium with lesser concentration of ingredients (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), MgSO₄.7 H₂O 0.0050 (g/l), CuSO₄ 0.0010 (g/l), FeSO₄ 0.0016 (g/l).

Thus the novel medium (FM1) with lower concentrations of ingredients is more economical & reduces fermentation period from 72 hrs to 48 hrs for sporulation as compared to the conventional medium (Molasses-yeast extract).

### Example 5:

### Production of spores of Trichoderma species in 50 litre pilot fermentor using the FM1 medium.

Spores of *Trichoderma* species raised in Roux bottles containing solid FM1 medium of the composition as described in Examples 1 &3 above were eluted in Tween 80 (0.02 % in water). 50 litres of FM1 medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060 (g/l), MgSO₄.7 H₂O 0.0050 (g/l), CuSO₄ 0.0010 (g/l), FeSO₄ 0.0016 (g/l) as described in Examples 1 &3 along with 15-25ml of silicon oil as antifoam (the amount of silicon oil varied depending upon development of foam during fermentation) was sterilised *in-situ* in the 100 1 fermentor (Andel, India) at 121°C /15 psig for 15 min. The medium on cooling was inoculated with 450-550 ml of spore suspension in Tween 80 solution with the final spores concentration of 1 x 10⁶/ml.

The fermentor was set with the following parameters:

| | |
|---|---|
| Agitation | = 250 rpm (revolutions per min.) |
| Aeration rate | = 0.5 vvm (volume per volume per min.) |
| Temperature | = 29°C ± 1 |
| Pressure | = 8.3 kPag (1.2 psig) |

The fermentation was continued for 48 hours until the spore concentration of 2.3 x 10⁸ was achieved (Table 5). Periodic sampling for spore counting was carried out at regular intervals of time as shown in the Table 5.

**Table 5**

| **Growth and sporulation** of *Trichoderma* species in **50-L fermentor using new FM1 medium** | | |
|---|---|---|
| **Fermentation Period (h)** | **Dry biomass (g/l)** | **Spore count/ml** |
| 0 | 0.02 | 1.0x10⁶ |
| 12 | 0.24 | - |
| 24 | 0.43 | 2.0x10⁵ |
| 30 | 0.31 | 3.7x10⁵ |
| 36 | 0.31 | 7.9x10⁶ |
| 42 | 0.34 | 4.9x10⁷ |
| 48 | 0.32 | 2.3x10⁸ |

### Example 6

### Production of spores of Trichoderma species in 50 litre pilot fermentor using FM1 medium with higher concentration of ingredients.

Spores of *Trichoderma* species raised in Roux bottles containing solid FM1 medium of the composition as described in Examples 1,3 &5 above were eluted in Tween 80 (0.02 % in water) solution as described in examples 1,3 and 5 above. 50 litres of FM1 medium with following ingredients Molasses 20 (g/l), Corn Steep Liquor (CSL) 25.00 (g/l), Sodium chloride 15.00 (g/l), CaSO₄ 0.500 (g/l), KH₂PO₄ 0.01 (g/l), MgSO₄.7 H₂O 0.01 (g/l), CuSO₄ 0.0050 (g/l), FeSO₄ 0.005 (g/l) without agar, along with 15-25ml of silicon oil as antifoam (the amount of silicon oil varied depending upon development of foam during fermentation) was sterilised *in-situ* in the 100 I fermentor (Andel, India) at 121°C/103 kPag (15 psig) for 15 min. The medium on cooling was inoculated with 450-550 ml of spore suspension in Tween 80 solution with the final spores concentration of 1 x 10⁶/ml.

The fermentor was set with the following parameters:

| | |
|---|---|
| Agitation | = 250 rpm (revolutions per min.) |
| Aeration rate | = 0.5 vvm (volume per volume per min.) |
| Temperature | = 29°C ± 1 |
| Pressure | = 8.3 kPag (1.2 psig) |

The fermentation was continued for 48 hours until the spore concentration of 2.3 x 10⁸ was achieved (Table 6). Periodic sampling for spore counting was carried out at regular intervals of time as shown in the Table 6.

**Table 6**

| **Growth and sporulation** of *Trichoderma* species **50 L fermentor using FM1 medium with higher concentration of ingredients.** | | |
|---|---|---|
| **Fermentation Period (h)** | **Dry biomass (g/l)** | **Spore count/ml** |
| 0 | 0.02 | 1.0x10⁶ |
| 12 | 0.26 | - |
| 24 | 0.48 | 2.5x10⁵ |
| 30 | 0.38 | 3.9x10⁵ |
| 36 | 0.39 | 8.2x10⁶ |
| 42 | 0.35 | 5.0x10⁷ |
| 48 | 0.30 | 2.7x10⁸ |

Perusal of the data show that the novel medium (FM1) with lower concentrations of ingredients is more economical and has the same spore concentration/ml and incubation period as the FM1 medium with higher concentration of ingredients.
Further the FM1 medium is far superior than the conventional medium (Molasses-yeast extract medium containing Molasses 7.50 (g/l), Glycerin 7.50 (g/l), Sodium Chloride 10.00 (g/l), Yeast Extract 5.00 (g/l), CaSO₄ 0.25 (g/l), KH₂PO₄ 0.006 (g/l), MgSO₄.7 H₂O 0.005 (g/l), CuSO₄ 0.001 (g/l), and Trace Elements 0.0016 (g/l) when spore concentration/ml and incubation period is taken into consideration.

### Advantages of the instant Application:

1. The composition of instant application facilitates early and profuse sporulation.
2. The said composition shows about 100-fold increase in spore count within 48 hours of inoculation.
3. The said composition is very economical as compared to other commercially available compositions for similar use.
4. The method of early and profuse sporulation in fungi facilitates easy availability of scantily sporulating fungi.
5. The said composition and method show significant utility for fungi like *Trichoderma* species, *Aspergillus* species, *Penicillium* species.
6. The major constituents of the medium for the mass production of *Trichoderma* species, a number of other sporulating ascomycetous fungi such as *Aspergillus* species, *Penicillium* species and any other scantily sporulating species of fungi under solid state, surface and submerged fermentation conditions, are inexpensive agro-based industrial by-products like beet-root pulp, wheat bran, corn-steep liquor and molasses.
7. The designed medium FM1 (medium containing Molasses 7.50 (g/l), Corn Steep Liquor (CSL) 20.00 (g/l), Sodium chloride 10.00 (g/l), CaSO₄ 0.2500 (g/l), KH₂PO₄ 0.0060(g/l), MgSO₄.7 H₂O 0.0050 (g/l), CuSO₄ 0.0010 (g/l), FeSO₄ 0.0016 (g/l) is not only inexpensive but results in profuse sporulation of the order of 200-300 million coniodiospores (2-3 x10⁸ spores/ml) of *Trichoderma* species, a number of other sporulating ascomycetous fungi such as *Aspergillus* species, *Penicillium* species and any other scantily sporulating species of fungi under solid state, surface and submerged fermentation conditions.
8. This novel medium (FM1) reduces fermentation period from 72 hrs in conventional medium to 48 hrs for sporulation. Moreover spore count (3.0x10⁸) is achieved in this novel medium which is 100 times from the initial inoculum (4-5 x I 0⁶) in 48 hrs whereas in case of conventional medium (Molasses-yeast) it is less than 10 times (1.05x10⁷) in 72 hrs. Besides, number of chlamydospores in conventional medium (Molasses-yeast) is much higher as compared to the novel medium (FM1) at any fixed time internal.
9. The spores of this biocontrol agent can be directly blended with the carrier agent without any further processing and used directly in the fields.

## Claims

1. A synergistic composition useful for early and profuse sporulation in fungi, said composition comprising molasses ranging between 5-20 g/Lm, Corn Steep Liquor (CSL) ranging between 10-25 g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄. 7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent.

2. A composition as claimed in claim 1, comprising molasses in an amount of 7.50 g/L, Corn Steep Liquor (CSL) in an amount of 20 g/L, Sodium chloride (NaCl) in an amount of 10 g/L, calcium sulphate (CaSO₄) in an amount of 0.25 g/L, Potassium dihydrogen phosphate (KH₂PO₄) in an amount of 0.0060 g/L, Magnesium sulphate (MgSO₄.7 H₂O) in an amount of 0.0050 g/L, Copper sulphate (CuSO₄) in an amount of 0.0010 g/L, Ferrous sulphate (FeSO₄) in an amount of 0.0016 g/L, an anti-foam agent, and optionally solidifying agent.

3. A composition as claimed in claim 1, wherein silicon oil is used as an anti-foam agent.

4. A composition as claimed in claim 3, wherein the silicon oil ranges between 0.1-0.5 ml.

5. A composition as claimed in claim 1, wherein the agar is used as a solidifying agent.

6. A composition as claimed in claim 5, wherein the agar ranges between 5-25 g/L.

7. A composition as claimed in claim 1, wherein said composition is used for mass production of fungi of biocontrol activity.

8. A use of a synergistic composition for early and profuse sporulation in fungi with about 100 fold increase in spore count within 48 hours of inoculation, said use comprising:
(a) inoculating about one week old spore culture of the fungi in a medium comprising molasses ranging between 5-20 g/L, Corn Steep Liquor (CSL) ranging between 10-25 g/L, Sodium chloride (NaCl) ranging between 5-15 g/L, calcium sulphate (CaSO₄) ranging between 0.1-0.5 g/L, Potassium dihydrogen phosphate (KH₂PO₄) ranging between 0.001-0.01 g/L, Magnesium sulphate (MgSO₄.7H₂O) ranging between 0.001-0.01 g/L, Copper sulphate (CuSO₄) ranging between 0.001-0.0050 g/L, Ferrous sulphate (FeSO₄) ranging between 0.0009-0.005 g/L, an anti-foam agent, and optionally solidifying agent.
(b) fermenting the inoculated culture at temperature about 27-30°C, and
(c) obtaining a large quantity of viable spores.

9. A use as claimed in claim 8, wherein said culture of step (a) is inoculated with said spores of final concentration of 1x10⁶ conidia/ml.

10. A use as claimed in claim 8, wherein said culture of step (b) is fermented under solid state, surface, or submerged fermentation conditions.

11. A use as claimed in claim 8, wherein agar is used as a solidifying agent.

12. A use as claimed in claim 11, wherein the agar ranges between 5-25 g/L.

13. A use as claimed in claim 8, wherein said inoculated culture is rotated in a rotor at a rate of 200 to 400 revolutions/minute.

14. A use as claimed in claim 13, wherein said culture is agitated with said rotor at 1.5 - 3.5 cm throw.

15. A use as claimed in claim 8, wherein said culture is fermented with an aeration rate of 0.5-1.0 wm (v/v min⁻¹).

16. A use as claimed in claim 8, wherein said culture is fermented with a pressure of 4.8-9.6 kPag (0.7-1.4 psig).

17. A use as claimed in claim 8, for mass production of fungi of biocontrol activity.

18. A use as claimed in claim 17, wherein said culture is mixed with a carrier in a ratio ranging between 1:2 to 2:1, to develop a commercial formulation with biocontrol activity.

19. A use as claimed in claim 18, wherein the carrier used is lignite.

20. A use as claimed in claim 8, wherein said medium comprises molasses in an amount of 7.50 g/L, Corn Steep Liquor (CSL) in an amount of 20 g/L, Sodium chloride (NaCl) in an amount of 10 g/L, calcium sulphate (CaSO₄) in an amount of 0.25 g/L, Potassium dihydrogen phosphate (KH₂PO₄) in an amount of 0.0060 g/L, Magnesium sulphate (MgSO₄.7 H₂O) in an amount of 0.0050 g/L, Copper sulphate (CuSO₄) in an amount of 0.0010 g/L, Ferrous sulphate (FeSO₄) in an amount of 0.0016 g/L, an anti-foam agent, and optionally solidifying agent.

21. A use as claimed in claim 8, wherein silicon oil is used as an anti-foam agent.

22. A use as claimed in claim 8, wherein the silicon oil ranges between 0.1-0.5 ml.

23. A use as claimed in claim 8, wherein fungi are selected from the group consisting of *Trichoderma* species, *Aspergillus* species and *Penicillium* species.

24. A use as claimed in claim 8, wherein fungi used are *Trichoderma* species.

## Patentansprüche

1. Synergistische Zusammensetzung für frühe und reichliche Sporenbildung bei Pilzen, wobei die Zusammensetzung Melasse im Bereich zwischen 5 und 20 g/l, Maisquellwasser (CSL) im Bereich zwischen 10 und 25 g/l, Natriumchlorid (NaCl) im Bereich zwischen 5 und 15 g/l, Calciumsulfat (CaSo₄) im Bereich zwischen 0,1 und 0,5 g/l, Kaliumdihydrogenphosphat (KH₂PO₄) im Bereich zwischen 0,001 und 0,01 g/l, Magnesiumsulfat (MgSO₄·7H₂O) im Bereich zwischen 0,001 und 0,01 g/l, Kupfersulfat (CuSO₄) im Bereich zwischen 0,001 und 0,0050 g/l, Eisen(II)-sulfat (FeSO₄) im Bereich zwischen 0,0009 und 0,005 g/l, ein Antischaummittel und wahlweise ein Verfestigungsmittel aufweist.

2. Zusammensetzung nach Anspruch 1, die Melasse in einem Anteil von 7,50 g/l, Maisquellwasser (CSL) in einem Anteil von 20 g/l, Natriumchlorid (NaCl) in einem Anteil von 10 g/l, Calciumsulfat (CaSo₄) in einem Anteil von 0,25 g/l, Kaliumdihydrogenphosphat (KH₂PO₄) in einem Anteil von 0,0060 g/l, Magnesiumsulfat (MgSO₄·7H₂O) in einem Anteil von 0,0050 g/l, Kupfersulfat (CuSO₄) in einem Anteil von 0,0010 g/l, Eisen(II)-sulfat (FeSO₄) in einem Anteil von 0,0016 g/l, ein Antischaummittel und wahlweise ein Verfestigungsmittel aufweist.

3. Zusammensetzung nach Anspruch 1, wobei Siliconöl als Antischaummittel verwendet wird.

4. Zusammensetzung nach Anspruch 3, wobei die Siliconölmenge im Bereich zwischen 0,1 und 0,5 ml liegt.

5. Zusammensetzung nach Anspruch 1, wobei Agar als Verfestigungsmittel verwendet wird.

6. Zusammensetzung nach Anspruch 5, wobei der Agaranteil im Bereich zwischen 5 und 25 g/l liegt.

7. Zusammensetzung nach Anspruch 1, wobei die Zusammensetzung für die Massenproduktion von Pilzen mit Wirksamkeit in der biologischen Schädlingsbekämpfung verwendet wird.

8. Anwendung einer synergistischen Zusammensetzung für frühe und reichliche Sporenbildung bei Pilzen mit etwa 100-fachem Anstieg der Sporenzahl innerhalb von 48 Stunden nach der Inokulation, wobei die Anwendung aufweist:
(a) Inokulieren einer etwa eine Woche alten Sporenkultur der Pilze in ein Medium, das Melasse im Bereich zwischen 5 und 20 g/l, Maisquellwasser (CSL) im Bereich zwischen 10 und 25 g/l, Natriumchlorid (NaCl) im Bereich zwischen 5 und 15 g/l, Calciumsulfat (CaSo₄) im Bereich zwischen 0,1 und 0,5 g/l, Kaliumdihydrogenphosphat (KH₂PO₄) im Bereich zwischen 0,001 und 0,01 g/l, Magnesiumsulfat (MgSO₄·7H₂O) im Bereich zwischen 0,001 und 0,01 g/l, Kupfersulfat (CuSO₄) im Bereich zwischen 0,001 und 0,0050 g/l, Eisen(II)-sulfat (FeSO₄) im Bereich zwischen 0,0009 und 0,005 g/l, ein Antischaummittel und wahlweise ein Verfestigungsmittel aufweist,
(b) Fermentieren der inokulierten Kultur bei einer Temperatur von etwa 27-30°C, und
(c) Gewinnung einer großen Zahl keimfähiger Sporen.

9. Anwendung nach Anspruch 8, wobei die Kultur von Schritt (a) mit den Sporen in einer Endkonzentration von 1 × 10⁶ Konidien/ml inokuliert wird.

10. Anwendung nach Anspruch 8, wobei die Kultur von Schritt (b) unter Feststoff-, Oberflächen- oder submersen Fermentierungsbedingungen fermentiert wird.

11. Anwendung nach Anspruch 8, wobei Agar als Verfestigungsmittel verwendet wird.

12. Anwendung nach Anspruch 11, wobei der Agaranteil im Bereich zwischen 5 und 25 g/l liegt.

13. Anwendung nach Anspruch 8, wobei die inokulierte Kultur in einem Rotor mit einer Drehzahl von 200 bis 400 Umdrehungen/Minute in Rotation versetzt wird.

14. Anwendung nach Anspruch 13, wobei die Kultur mit dem Rotor mit einer Exzentrizität von 1,5-3,5 cm gerührt wird.

15. Anwendung nach Anspruch 8, wobei die Kultur mit einer Belüftungsrate von 0,5-1,0 vvm (Volumen/Volumen·Minute⁻¹) fermentiert wird.

16. Anwendung nach Anspruch 8, wobei die Kultur mit einem Druck von 4,8-9,6 kPa (ü) (0,7-1,4 psig) fermentiert wird.

17. Anwendung nach Anspruch 8 zur Massenproduktion von Pilzen mit Wirksamkeit in der biologischen Schädlingsbekämpfung.

18. Anwendung nach Anspruch 17, wobei die Kultur in einem Verhältnis im Bereich von 1:2 bis 2:1 mit einem Träger vermischt wird, um eine kommerzielle Formulierung mit Wirksamkeit in der biologischen Schädlingsbekämpfung zu entwickeln.

19. Anwendung nach Anspruch 18, wobei der Träger Lignit ist.

20. Anwendung nach Anspruch 8, wobei das Medium Melasse in einem Anteil von 7,50 g/l, Maisquellwasser (CSL) in einem Anteil von 20 g/l, Natriumchlorid (NaCl) in einem Anteil von 10 g/l, Calciumsulfat (CaSo₄) in einem Anteil von 0,25 g/l, Kaliumdihydrogenphosphat (KH₂PO₄) in einem Anteil von 0,0060 g/l, Magnesiumsulfat (MgSO₄·7H₂O) in einem Anteil von 0,0050 g/l, Kupfersulfat (CuSO₄) in einem Anteil von 0,0010 g/l, Eisen(II)-sulfat (FeSO₄) in einem Anteil von 0,0016 g/l, ein Antischaummittel und wahlweise ein Verfestigungsmittel aufweist.

21. Anwendung nach Anspruch 8, wobei Siliconöl als Antischaummittel verwendet wird.

22. Anwendung nach Anspruch 8, wobei die Siliconölmenge im Bereich zwischen 0,1 und 0,5 ml liegt.

23. Anwendung nach Anspruch 8, wobei Pilze aus der Gruppe ausgewählt sind, die aus der Spezies Trichoderma, der Spezies Aspergillus und der Spezies Penicillum besteht.

24. Anwendung nach Anspruch 8, wobei die verwendeten Pilze die Spezies Trichoderma sind.

## Revendications

1. Composition synergétique utile pour la sporulation précoce et profuse chez les champignons, ladite composition comprenant des mélasses s'étendant entre 5 à 20 g/L, de l'eau de trempage de maïs (CSL) en une teneur s'étendant entre 10 à 25 g/L, du chlorure de sodium (NaCl) en une teneur s'étendant entre 5 à 15 g/L, du sulfate de calcium (CaSO₄) en une teneur s'étendant entre 0,1 à 0,5 g/L, du dihydrogénophosphate de potassium (KH₂PO₄) en une teneur s'étendant entre 0,001 à 0,01 g/L, du sulfate de magnésium (MgSO₄•7H₂O) en une teneur s'étendant entre 0,001 à 0,01 g/L, du sulfate de cuivre (CuSO₄) en une teneur s'étendant entre 0,001 à 0,0050 g/L, du sulfate ferreux (FeSO₄) en une teneur s'étendant entre 0,0009 à 0,005 g/L, un agent anti-mousse, et de manière facultative un agent solidifiant.

2. Composition selon la revendication 1, comprenant des mélasses en une quantité de 7,50 g/L, de l'eau de trempage de maïs (CSL) en une quantité de 20 g/L, du chlorure de sodium (NaCl) en une quantité de 10 g/L, du sulfate de calcium (CaSO₄) en une quantité de 0,25 g/L, du dihydrogénophosphate de potassium (KH₂PO₄) en une quantité de 0,0060 g/L, du sulfate de magnésium (MgSO₄•7H₂O) en une quantité de 0,0050 g/L, du sulfate de cuivre (CuSO₄) en une quantité de 0,0010 g/L, du sulfate ferreux (FeSO₄) en une quantité de 0,0016 g/L, un agent anti-mousse, et de manière facultative un agent solidifiant.

3. Composition selon la revendication 1, dans laquelle de l'huile de silicone est utilisée comme agent anti-mousse.

4. Composition selon la revendication 3, dans laquelle la teneur en huile de silicone s'étend entre 0,1 à 0,5 ml.

5. Composition selon la revendication 1, dans laquelle de l'agar-agar est utilisé comme agent solidifiant.

6. Composition selon la revendication 5, dans laquelle la teneur en agar-agar s'étend entre 5 à 25 g/L.

7. Composition selon la revendication 1, dans laquelle ladite composition est utilisée pour la production en masse de champignons présentant une activité de contrôle biologique.

8. Utilisation d'une composition synergétique pour la sporulation précoce et profuse chez les champignons avec une augmentation de la numération des spores d'environ 100 fois dans les 48 heures de l'inoculation, ladite utilisation comprenant:
(a) l'inoculation d'une culture de spores âgée d'environ une semaine des champignons dans un milieu comprenant des mélasses en une teneur s'étendant entre 5 à 20 g/L, de l'eau de trempage de maïs (CSL) en une teneur s'étendant entre 10 à 25 g/L, du chlorure de sodium (NaCl) en une teneur s'étendant entre 5 à 15 g/L, du sulfate de calcium (CaSO₄) en une teneur s'étendant entre 0,1 à 0,5 g/L, du dihydrogénophosphate de potassium (KH₂PO₄) en une teneur s'étendant entre 0,001 à 0,01 g/L, du sulfate de magnésium (MgSO₄•7H₂O) en une teneur s'étendant entre 0,001 à 0,01 g/L, du sulfate de cuivre (CuSO₄) en une teneur s'étendant entre 0,001 à 0,0050 g/L, du sulfate ferreux (FeSO₄) en une teneur s'étendant entre 0,0009 à 0,005 g/L, un agent anti-mousse, et de manière facultative un agent solidifiant.
(b) la fermentation de la culture inoculée à une température d'environ 27 à 30°C, et
(c) l'obtention d'une grande quantité de spores viables.

9. Utilisation selon la revendication 8, dans laquelle ladite culture de l'étape (a) est inoculée avec lesdits spores d'une concentration finale de 1 x 10⁶ conidies/ml.

10. Utilisation selon la revendication 8, dans laquelle ladite culture de l'étape (b) est fermentée à l'état solide, en surface, ou dans des conditions de fermentation avec submersion.

11. Utilisation selon la revendication 8, dans laquelle de l'agar-agar est utilisé comme agent solidifiant.

12. Utilisation selon la revendication 11, dans laquelle la teneur de l'agar-agar s'étend entre 5 à 25 g/L.

13. Utilisation selon la revendication 8, dans laquelle ladite culture inoculée est mise en rotation dans un rotor à un taux de 200 à 400 révolutions/minute.

14. Utilisation selon la revendication 13, dans laquelle ladite culture est agitée avec ledit rotor avec une excentricité de 1,5 à 3,5 cm.

15. Utilisation selon la revendication 8, dans laquelle ladite culture est fermentée avec un taux d'aération de 0,5 à 1,0 vvm (vol/vol.min⁻¹).

16. Utilisation selon la revendication 8, dans laquelle ladite culture est fermentée avec une pression de 4,8 à 9,6 kPag (0,7 à 1,4 lb/po²).

17. Utilisation selon la revendication 8, pour la production en masse de champignons présentant une activité de contrôle biologique.

18. Utilisation selon la revendication 17, dans laquelle ladite culture est mélangée avec un support en un rapport s'étendant entre 1:2 jusqu'à 2:1, pour développer une formulation commerciale présentant une activité de contrôle biologique.

19. Utilisation selon la revendication 18, dans laquelle le support utilisé est la lignite.

20. Utilisation selon la revendication 8, dans laquelle ledit milieu comprend des mélasses en une quantité de 7,50 g/L, de l'eau de trempage de maïs (CSL) en une quantité de 20 g/L, du chlorure de sodium (NaCl) en une quantité de 10 g/L, du sulfate de calcium (CaSO₄) en une quantité de 0,25 g/L, du dihydrogénophosphate de potassium (KH₂PO₄) en une quantité de 0,0060 g/L, du sulfate de magnésium (MgSO₄•7H₂O) en une quantité de 0,0050 g/L, du sulfate de cuivre (CuSO₄) en une quantité de 0,0010 g/L, du sulfate ferreux (FeSO₄) en une quantité de 0,0016 g/L, un agent anti-mousse, et de manière facultative un agent solidifiant.

21. Utilisation selon la revendication 8, dans laquelle de l'huile de silicone est utilisée comme agent anti-mousse.

22. Utilisation selon la revendication 8, dans laquelle la teneur en huile de silicone s'étend entre 0,1 à 0,5 ml.

23. Utilisation selon la revendication 8, dans laquelle les champignons sont choisis parmi le groupe constitué des espèces du genre *Trichoderma*, des espèces du genre *Aspergillus* et des espèces du genre *Penicillium*.

24. Utilisation selon la revendication 8, dans laquelle les champignons utilisés sont des espèces du genre *Trichoderma*.
